# EUROPEAN PATENT APPLICATION

(11) **EP 3 733 195 A1**
(43) Date of publication of application: **04.11.2020**
(21) Application number: 20172591.8
(22) Date of filing: 01.05.2020
(51) Int. Cl.: A61K 36/324, A61P 35/00

(54) **BOSWELLIC ACID EXTRACTION**

(30) Priority: 02.05.2019 ZA 201902740
(71) Applicant: Dhofar University, Salalah (OM)
(72) Inventor: RASHAN, Luay, 123 Al-Khod (OM); HASSON, Sidgi Syed Anwer Abdo, 123 Al-Khod (OM); SIMMET, Thomas, 123 Al-Khod (OM); AL BALUSHI, Mohammed Said Amur, 123 Al-Khod (OM); AL JABRI, Ali Abdullah Hasan, 123 Al-Khod (OM)
(74) Representative: Mewburn Ellis LLP

(57) **Abstract**

The invention is directed to an improved method of extracting boswellic acid from Boswellia gum resin and the use of extracted boswellic acid in the manufacture of a pharmaceutical composition for treatment of an illness or disease in a patient in need thereof.

## Description

THIS INVENTION relates to an improved, cost effective, eco-friendly method for the extraction of boswellic acid from the waste product residues of the Omani *Boswellia sacra* gum resin. The method results in a yield that is with over 70% higher than that produced using conventional methods.

Plant-derived drugs are an important resource to combat diseases, particularly in developing countries. Approximately 60-80% of the world's population is reliant on traditional medicine for the treatment of common illnesses and ailments. Approximately 60-90% of patients with arthritis who have used complementary and alternative medicine, have used traditional Chinese medicine. For at least 3000 years, Olibanum, also known as frankincense, had been an important trade item for the civilizations located in the Arabian Peninsula and in North Africa, such as Somalia, particularly due to its use as an incense material.

Olibanum is a natural oleo-gum-resin that is obtained through incisions made in the trunks of trees of the genus Boswellia. The genus Boswellia belongs to the Burseraceae family and includes several species that grow in a range of countries in the Arabian Pensinsula, India and East Africa and is represented by approximately 43 different species of trees and shrubs. This includes *Boswellia serrata* in India, *Boswellia carterri* in East Africa and China, *Boswellia frereana* in Somalia, and *Boswellia sacra* in Yemen and Oman, each producing a slightly different type of resin. Differences in soil and climate create more diversity in the resins, even within the same species. On the island of Soqotra in Yemen, a unique and endemic flora occurs as a result of its long geological isolation and the present hot and dry climate conditions, including eight endemic species from the genus Boswellia.

Crude Boswellia gum resin is a mixture of different terpenoids, polysaccharides, and inorganic salts. The triterpenoid fraction of the gum contains certain active compounds responsible for its pharmacological activities. These compounds include pentacyclic triterpenic acids that are commonly known as boswellic acids. In the last two decades, Olibanum has gained increasing attention from scientists and pharmaceutical companies to better define its medical effects. Several studies have reported on the anticancer, antiinflammatory, immunomodulatory, antimicrobial and antiviral activities of several Boswellia species. However, Boswellia extracts are being marketed under high dosage forms for the management of such clinical complications with moderate to high side effects. These extracts contain boswellic acids as the active compounds, including beta-boswellic acid, acetyl-beta-boswellic acid, 11 -keto-beta-boswellic acid, acetyl- 11-keto-Boswellic acid, alpha-boswellic acid and acetyl-alpha-boswellic acid. The boswellic acids content in commercially available Boswellia gum extract generally varies between 50-90% by weight estimated as total organic acids. These extracts are being prepared using various organic solvents that have potential health risks due to their toxicity and hazardous properties.

The invention provides a composition comprising acetyl-11-keto-beta-boswellic acid (AKBA) and a composition comprising 11-keto-beta-boswellic acid (KBA) in combination with one or more components selected from biologically active ingredients, functional ingredients, excipients, diluents, carriers and additives or mixtures thereof.

The invention also provides a synergistic composition comprising Boswellia low polar gum resin extract (BLPRE) that is obtained from the waste product residues that are a product of a method of heavy extraction of crude gum resin. This method of heavy extraction includes dissolving an aqueous mixture of boswellic acids to extract a volatile oil and other components by hydro-distillation. The waste product residue is immediately stored for the novel extraction purpose. The composition can be used at lower concentration with less side effects, if any, compared to boswellic acids that are conventionally extracted. The composition can be used for the alleviation of numerous inflammatory conditions, including mild to moderate kidney dysfunction, motor neuron disease, peripheral neuropathy, cancers including glioblastoma multiforme, glioma, ovarian, breast, and inflammatory diseases linked to immune dysfunction. The composition can be administered by parenteral, oral, injectable or topical means.

The present invention provides an improved method for the extraction of boswellic acids from the waste product residues of *Boswellia sacra* gum resin.

In an embodiment, the present invention provides a pharmaceutical composition comprising at least one standardized fraction of boswellic acid obtained by the method with at least one pharmaceutically acceptable excipient. It is also directed to the use of the pharmaceutical composition for the treatment and management of treating mild, moderate kidney dysfunction; diseases including motor neuron disease and peripheral neuropathy; cancers including glioblastoma multiforme, glioma, ovarian, and breast cancer; and inflammatory diseases linked to the immune dysfunctions. The composition is for parenteral, oral, injectable or topical administration, such as for eczema. The invention further provides a method for treating the abovementioned diseases and disorders by administering the pharmaceutical composition to the subject.

According to a first aspect of the invention, there is provided a method of extracting boswellic acid from Boswellia gum resin, which may include the steps of: obtaining a residue by extracting Boswellia oil from Boswellia gum resin; precipitating the residue and separating the precipitate; washing and freezing the precipitate; grinding the frozen precipitate to a powder; drying the powder and extracting the boswellic acid using ethanol; and purifying the extracted boswellic acid.

The Boswellia gum resin may be ground to a powder in order to extract the oil by hydro-distillation.

The precipitate may be washed with distilled water.

The powder may be dried at a temperature of at least 80°C.

The extracted boswellic acid may be purified by isolating a supernatant layer from the extracted boswellic acid and drying the supernatant to obtain a solid.

The boswellic acid may be extracted from *Boswellia sacra, Boswellia serrata,* or *Boswellia frereana.*

According to a second aspect of the invention, there is provided a use of the extracted boswellic acid obtained by the first aspect of the invention, and a pharmaceutically acceptable excipient in the manufacture of a pharmaceutical composition for treatment of an illness or disease in a patient in need thereof.

The illness or disease may be selected from kidney dysfunction, motor neuron disease, peripheral neuropathy, cancer (such as includes glioblastoma multiforme, glioma, ovarian cancer and breast cancer), inflammatory disease, and eczema.

The invention will now be described in more detail, with reference to the following nonlimiting Example and the accompanying drawing.

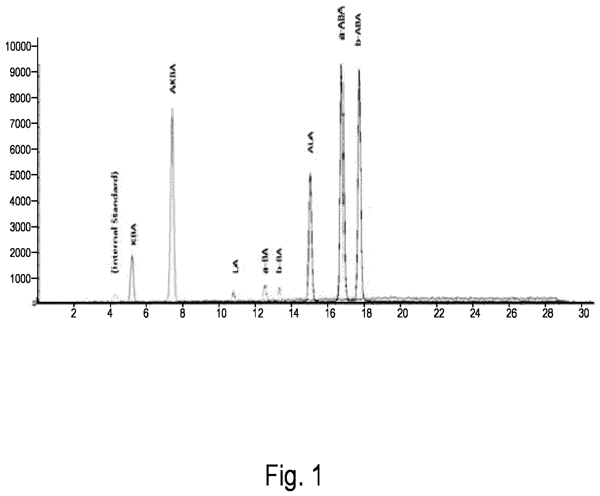

In the drawings
FIGURE 1 shows, for the Example, a fraction analysis of extracted samples.

### EXAMPLE

### Materials and Methods

### Preparation of standardized fractions of boswellic acids from gum resin waste product of Boswellia sacra and their purification.

250 mg of oleo gum resin (Najdi cultivar) is stored in a deep freeze (-10, -20, 80 °C) for a period of 1-12 hours. The gum resin is crushed using an electrical grinder to obtain a fine powder. The essential oil is obtained by hydro-distillation after adding 2.5 I of distilled water for a period of between 1-8 hours using a Cleavinger.

The residue is stored in a refrigerator for a period of 1-24 hours. An off-white precipitate is obtained. This is separated by filtration using filter Whatman filter papers No.1 and 3 to give a yield of 190 g. The precipitate is washed several times with cold distilled water and stored in a deep freeze for a period of 1-12 hours . The precipitate is crushed using an electrical grinder to obtain a fine powder. The powder is dried in an oven at a temperature above 80°C under rotary evaporator to obtain the standardized boswellic acid fractions.

The granulated fine powder is extracted using 100% ethanol with continuous stirring using a magnetic stirrer for a period of 1-48 hours under room temperature. The supernatant layer is separated using a separating funnel and dried under high vacuum pressure using a rotary evaporator to obtain a solid material with a yield of 162 g. The extract is diluted with water and precipitated with saline or with an inorganic acid at pH 7.0.

### Analysis of Boswellia samples by HPLC-MS/MS (Table 2)

1 mg of the prepared sample is mixed with 1 ml 100% MeOH and subjected to 5 minutes of ultrasound. A 30 minutes extraction is performed at RT with continuous stirring. The mixture is centrifuged at 16,000 g for 5 minutes. A 1:10 and 1:100 dilution of the mixture is prepared. The undiluted and diluted mixtures are subjected to reverse phase HPLC and tandem MS (MRM) using HPLC-MS/MS in order to quantify the boswellic acids.

### Results and Discussion

The present invention provides a method for the preparation and purification of standardized fractions of boswellic acid to produce a high yield of boswellic acid. The method is ecofriendly, cost effective and technically simple, avoiding the use of additives and toxic solvents such as hexanes, ethers, ketones, esters, chlorinated hydrocarbons.

**Table 1: Analysis of extracted samples from waste product gum resin by HPLC-MS/MS**

| **Substances** | **Tr [min]** |
|---|---|
| 11-Keto-β-boswellic acid (KBA) | 4.9 |
| Acetyl-11-keto-β-boswellic acid (AKBA) | 7.0 |
| Lupeolic acid | 10.4 |
| α-Boswellic acid (a-BA) | 12.0 |
| B-Boswellic acid (b-BA) | 12.8 |
| Acetyl-lupeolic acid | 14.5 |
| Acetyl-α-boswellic acid (a-ABA) | 16.2 |
| Acetyl-β-boswellic acid (b-ABA) | 17.1 |

**Table 2: Sample composition analysis of extracted samples from waste product gum resin**

| **Sample** | **Concentration [µg/mg] (w/w)** | | | | | | | |
|---|---|---|---|---|---|---|---|---|
| | **KBA** | **AKBA** | **LA** | **a-BA** | **b-BA** | **ALA** | **a-ABA** | **b-ABA** |
| BS2 | 2.560 | 73.200 | 9.010 | 19.850 | 50.600 | 59.900 | 65.400 | 111.000 |
| BS3 | 3.245 | 67.400 | 8.305 | 21.800 | 59.350 | 55.000 | 66.700 | 113.500 |
| BS4 | 2.555 | 58.500 | 9.600 | 18.500 | 43.850 | 46.600 | 52.800 | 92.250 |
| BS5 | 0.040 | 0.654 | 0.121 | 0.283 | 0.639 | 0.582 | 0.800 | 1.335 |
| BS6 | 0.076 | 0.985 | 0.226 | 0.370 | 0.877 | 0.870 | 1.170 | 1.955 |
| BS7 | 3.320 | 50.400 | 8.660 | 14.850 | 40.250 | 42.550 | 49.900 | 86.200 |
| BS8 | 1.205 | 21.200 | 3.325 | 12.050 | 13.000 | 14.950 | 18.600 | 25.000 |
| LOD (DIN 32645) | 0.0043 | 0.0039 | 0.0195 | 0.0161 | 0.0158 | 0.0161 | 0.0088 | 0.0090 |
| LOQ (DIN 32645 | 0.0260 | 0.0145 | 0.0719 | 0.0601 | 0.0589 | 0.0600 | 0.0346 | 0.0340 |

Table 1 and Table 2 set out the results from HPLC-MS/MS. The standardized fractions of boswellic acid contain 50-70%, preferably 75% to 85%, of total boswellic acids by weight which is estimated as the total organic acids. The standardized boswellic acids fraction comprises BS2 (*B. sacra* extract in 100 % EtOH); BS3 (*B. sacra* extract in EtOH/H₂O [50/50]); BS4 (*B. sacra* extract in 100 % MeOH); BS5 (*B. sacra* extract in boiling H₂O); BS6 (*B. sacra* extract in H₂O at room temperature); BS7 (*B. sacra* extract after hydro-distillation); and BS8 (*B. sacra* extract supernatant after hydro-distillation).

## Claims

1. A method of extracting boswellic acid from Boswellia gum resin, the method comprising the steps of:
obtaining a residue by extracting Boswellia oil from Boswellia gum resin;
precipitating the residue and separating the precipitate;
washing and freezing the precipitate;
grinding the frozen precipitate to a powder;
drying the powder and extracting the boswellic acid using ethanol; and
purifying the extracted boswellic acid.

2. The method of claim 1, wherein the Boswellia gum resin is ground to a powder in order to extract the oil by distillation.

3. The method of claim 1 or 2, wherein the Boswellia oil is extracted by hydro-distillation.

4. The method of any one of the preceding claims, wherein the precipitate is washed with distilled water.

5. The method of any one of the preceding claims, wherein the powder is dried at a temperature of at least 80°C.

6. The method of any one of the preceding claims, wherein the extracted boswellic acid is purified by isolating a supernatant layer from the extracted boswellic acid and drying the supernatant to obtain a solid.

7. The method of any one of the preceding claims where the boswellic acid is extracted from *Boswellia sacra, Boswellia serrata,* or *Boswellia frereana.*

8. Use of an extracted boswellic acid obtained by the method of any one of claims 1 to 7 and a pharmaceutically acceptable excipient in the manufacture of a pharmaceutical composition for treatment of an illness or disease in a patient in need thereof.

9. The use of claim 8, wherein the illness or disease is at least one selected from kidney dysfunction, motor neuron disease, peripheral neuropathy, cancer, inflammatory disease, and eczema.

10. The use of claim 9, wherein the cancer includes glioblastoma multiforme, glioma, ovarian cancer and breast cancer.
